(19) Europäisches Patentamt
European Patent Office
Office européen des brevets

(11) Publication number: **0 088 008**
**B1**

(12) # EUROPEAN PATENT SPECIFICATION

(45) Date of publication of patent specification: **04.02.87**

(51) Int. Cl.[4]: **C 07 D 277/34**

(21) Application number: **83400349.3**

(22) Date of filing: **18.02.83**

(54) **Improved process for the production of 2-(4-(2-thiazolyloxy)phenyl)propionic acid.**

(30) Priority: **26.02.82 JP 31428/82**

(43) Date of publication of application:
**07.09.83 Bulletin 83/36**

(45) Publication of the grant of the patent:
**04.02.87 Bulletin 87/06**

(84) Designated Contracting States:
**AT BE CH IT LI NL SE**

(56) References cited:
**GB-A-1 481 465**

(73) Proprietor: **SHIONOGI & CO., LTD.**
**12, Dosho-machi 3-chome Higashi-ku**
**Osaka 541 (JP)**

(72) Inventor: **Hamada, Yoshinori**
**1-281, Hagiwaradai-nishi**
**Kawanishi-shi Hyogo Pref. (JP)**
Inventor: **Ando, Satoru**
**2-1812-24, Wakaba-cho**
**Oumihachiman-shi Shiga Pref. (JP)**

(74) Representative: **Hranitzky, Wilhelm Max et al**
**NOVOPAT-CABINET CHEREAU 5,**
**Place du Molard**
**CH-1204 Genève (CH)**

**EP 0 088 008 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to an improvement in the production of 2-[4-(2-thiazolyloxy)phenyl]propionic acid. More particularly, this invention provides a process for preparing the above phenylpropionic acid derivative in excellent yield on an industrial scale.

Thiazolyloxyphenylpropionic acids are useful compounds showing excellent anti-inflammatory and analgesic activities as noted in Japanese Patent Publication No. 1980/37556. Especially, 2-[4-(2-thiazolyloxy)phenyl]propionic acid is superior in the above pharmacological activities with low toxicity and the development of it as a medicine has been energetically carried out.

The inventors continued to develop a new improved process for the production and reached the process of the present invention. The process has the following advantages in the α-methylation of phenylacetic acid; easiness of the operation, use of readily available and handeable reagents, reduction of the cost, and the like.

There are many processes for preparing analgesics of phenylpropionic acid-type being represented by ibuprofen, fenoprofen, and the like. Representative of the processes consists of α-methylation of phenylacetic acid esters and subsequent hydrolysis of the ester group.

In general, a methyl halide and a base are combined to be used as a reagent for the methylation of the α position of phenylacetic acids; e.g. methyl iodide and sodium amide, methyl iodide and butyl lithium, and the like. The reagents, however, are not favorable since the bases are expensive and are not easily handled. Furthermore, it is noteworthy that methyl or ethyl esters of the phenylacetic acid derivatives which cause troubles in isolation and purification of the product after the hydrolysis are used as the starting material in the α-methylation. Any report has not been issued on the increase of the yield of the product by selection of the reaction conditions and the ester residue.

The objective compound, 2-[4-(2-thiazolyloxy)phenyl]propionic acid (abbreviated to as objective compound hereinafter) can be prepared by α-methylation of the corresponding phenylacetic acid derivative. Namely, the corresponding phenylacetic acid ester (I) is subjected to reaction with methyl halide in the presence of a condensing agent consisting of an alkali metal or of an alkali metal or earth-alkali metal compound selected from alkali metal alkoxides, magnesium alkoxides, alkali metal amides, alkylamino alkali metals, butyl or phenyl lithium, sodium hydride and triphenylmethyl sodium to give an α-phenylpropionic acid ester (II). Such a process is disclosed, for instance, in GB—A—1,481,465. Ordinary reaction, however, affords a mixture of α-phenylpropionic acid ester with the starting phenylacetic acid ester unreacted.

The separation of these compounds is very hard because of a very small difference in physical properties. Therefore, the obtained product cannot be used as it is for medical purpose in respect of low purity.

Furthermore, the methine at the α-position of the produced propionic acid ester is readily methylated to give a considerable amount of a by-product dimethylated derivative, α-phenylisobutyric acid ester (abbreviated to as dimethyl derivative hereinafter) when the methylation is continued until the starting phenylacetic acid ester is completely consumed. Separation of the ester of the objective compound from the dimethyl derivative is also difficult. The method cannot yield the product in the desired purity. Additionally, there is no difference between the ester of the objective compound and the above by-product in the susceptibility to hydrolysis, and it is still troublesome to separate the objective compound after hydrolysis.

Therefore, a method for preventing the occurrence of the dimethyl derivative was considered. The method consists of blocking of the methylene group of the acetic acid residue with a cyano or carboxy group to prevent further methylation to the dimethyl derivative, which has been shown in Japanese Patent Publication 1980/37556, practically illustrated in the examples 3 and 4. The method, however, invites a new trouble; i.e. cleavage of the ether bond between the thiazole ring and the benzene ring. Obviously, this extra step for protection and deprotection unavoidably invites decrease of the yield.

After extensive investigation directed to dissolving the above problem the inventors have developed a new process for preparing the objective compound in high yield by practising the α-methylation of phenylacetic acid in the following conditions:

(1) adopting isopropyl, sec-butyl, tert-butyl or benzhydryl ester of 4-(2-thiazolyloxy)phenylacetic acid;

(2) using methyl bromide as a reagent for methylation and powdered potassium hydroxide as a base;

(3) practising the methylation in dimethylformamide at −5 to 5°C; and, if necessary,

(4) effecting the subsequent hydrolysis of the ester with sodium hydroxide in an aqueous methanol, ethanol or isopropanol.

When the production of the objective compound is practised under the above conditions,

(a) the mono methylation, which does not occur with pellet potassium hydroxide, proceeds smoothly;

(b) the formation of the dimethyl derivative, α-phenylisobutyric acid ester is minimized, and furthermore the methylation can be practised thoroughly without regards to over-methylation because the dimethyl derivative can easily be removed at the following hydrolysis step;

(c) the dimethyl derivative can easily be removed from the objective compound since the ester residue of the dimethyl derivative is hardly hydrolyzed under such a condition of ester hydrolysis as noted in the above item (4); and

(d) the ether linkage between thiazole ring and benzene ring is not split.

Accordingly, the process of this invention provides the highly pure objective compound in excellent yield. Furthermore, the process has many merits such as minimization of multi-reaction steps, the simplified operations and use of the inexpensive reagents and no pollution problem.

The overall yield of the product by the methylation of the α position and the hydrolysis rises up to approximately 85% when the isopropyl ester is adopted while it is 76.90% when the methyl ester is used.

The detail of the process is explained below. The process consists of steps A and B and is illustrated by the following reaction scheme:

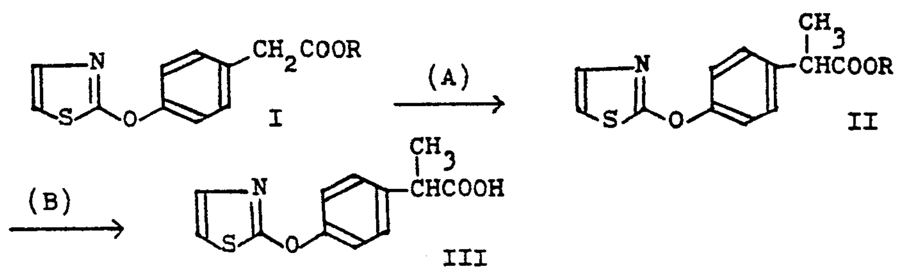


(wherein R is isopropyl, sec-butyl, tert-butyl or benzhydryl).

Step (A)

Isopropyl, sec-butyl, tert-butyl and benzhydryl ester of 2-[4-(2-thiazolyloxy)phenyl]acetic acid are used: the isopropyl, sec-butyl and benzhydryl esters are preferred; the isopropyl ester is most preferred.

An ester (I) of phenylacetic acid is added to a solution of powdered potassium hydroxide and methyl bromide in dimethylformamide and is allowed to react at −5 to 5°C, preferably at 0—5°C. Methyl bromide and potassium hydroxide are used in almost equal moles 1:1—1:0.8, preferably 1:1. The ratio of methyl bromide or potassium hydroxide to the phenylacetic acid ester is about 1.0—3.0 equivalent moles, preferably 1.5—2.5 equivalent moles, most preferably 2.0—2.5. The concentration of methyl bromide in dimethylformamide is 2.0—4.0 M/L, preferably 2.5—3.0 M/L.

The progress of the reaction is checked by high performance liquid chromatography (HPLC) and the reaction is stopped when the starting compound (I) disappears. The reaction may usually be finished within about 3 to 6 hours. The product (II) is isolated in the usual manner, for example, by extraction with organic solvents.

Step (B)

The ester (II) of α-phenylpropionic acid obtained in the above step (A) is hydrolyzed in an aqueous methanol, ethanol or isopropanol in the presence of sodium hydroxide.

The ratio of sodium hydroxide to α-phenylpropionic acid ester (II) is about 2—6 equivalent moles, preferably 2.5—4.5 equivalent moles, most preferably 4 equivalent moles. The aqueous alcoholic consists of water and an alcohol selected from methanol, ethanol and isopropanol, preferably methanol at a ratio of preferably 1:2—5 (v/v), especially 1:3. The concentration of sodium hydroxide in water is 11—25% (w/v), preferably 20—23% (w/v). The hydrolysis is effected at about 0—70°C, preferably 10—30°C, most preferably 15—25°C.

The reaction is traced by HPLC and the reaction is stopped when α-phenylpropionic acid ester (II) disappears usually 1 to 10 hours after, practically after 2 to 6 hours. The reaction mixture is extracted with a water-immisible organic solvent to remove the unhydrolyzed dimethyl derivative. The isolation of the objective compound is effected in the usual manner such as extraction, chromatography, and the like.

The product (III) obtained by the above process does not contain any starting material, an ester (I) of phenylacetic acid and contains less than 0.2% of the dimethyl derivative, an α-phenylisobutyric acid ester. Moreover, the purity is more than 99% and it can be improved up to almost 100% by recrystallization.

Besides, the starting compound (I) is prepared by transesterification of commercially available methyl 4-hydroxyphenylacetate to a desired ester and subsequent reaction of the ester with an 1-alkoxy-2-halogenethyl isothiocyanate followed by elimination of an alcohol according to the method disclosed in Japanese Patent Publication (Unexamined) 1979/109969.

The process of this invention is illustrated by the following examples.

Example 1

(A) Preparation of isopropyl 2-[4-(2-thiazolyloxy)phenyl]propionate

To a 2.62 M/L solution of methyl bromide in dimethylformamide (17.75 L) is added powdered potassium hydroxide (2400 g) with cooling under nitrogen atmosphere and then isopropyl 2-[4-(2-thiazolyloxy)phenyl]acetate (Ia) (5.156 kg) is added dropwise thereto. After stirred at 0—4°C for 4 hours, the mixture is adjusted to pH 3.0 with 40% sulfuric acid and then toluene (15 L) and water (50 L) are added thereto. The separated organic layer is washed with water and filtered after addition of active carbon (160 g). The residue is washed with toluene. The filtrate and the washings are combined and condensed under

reduced pressure to give an oil (6.43 kg) of isopropyl 2-[4-(2-thiazolyloxy)phenyl]propionate (IIa). The product is subjected to HPLC [with an eluent (acetonitrile:water:acetic acid = 50:50:0.1)] which indicates the product containing the following impurities:

| | |
|---|---|
| Starting material (Ia) | 0.00% |
| Dimethyl derivative (IIb)* | 5.94% |

*IIb = isopropyl 2-[4-(2-thiazolyloxy)phenyl]isobutyric acid

(B) Preparation of 2-[4-(2-thiazolyloxy)phenyl]propionic acid

The product (3.217 kg) of the above step (A) is added to methanol (16 L) and then an aqueous solution (5.4 L) of sodium hydroxide (1487 g) is added dropwise thereto under cooling. The mixture is stirred at 19—21°C for 2.5 hours, then adjusted to pH 7.0 by adding 40% sulfuric acid dropwise and condensed under reduced pressure. The residue is adjusted to pH 8.5 with a 10% sodium hydroxide solution and extracted with toluene. The aqueous layer is filtered after addition of active carbon (140 g) and to the filtrate is added toluene (2.5 L). The mixture is adjusted to pH 3.5 with 40% sulfuric acid and stirred at room temperature to precipitate the title compound, 2-[4-(2-thiazolyloxy)phenyl]propionic acid (2158 g), which is collected by filtration and recrystallized from ethylene dichloride to give crystals (1969 g) melting at 120—121.5°c with the purity of 99.94%. Overall yield is 84.95%.

Examples 2—4

The same steps as noted in Example 1 are carried out under the reaction conditions noted in the following table to give Compound III as follows:

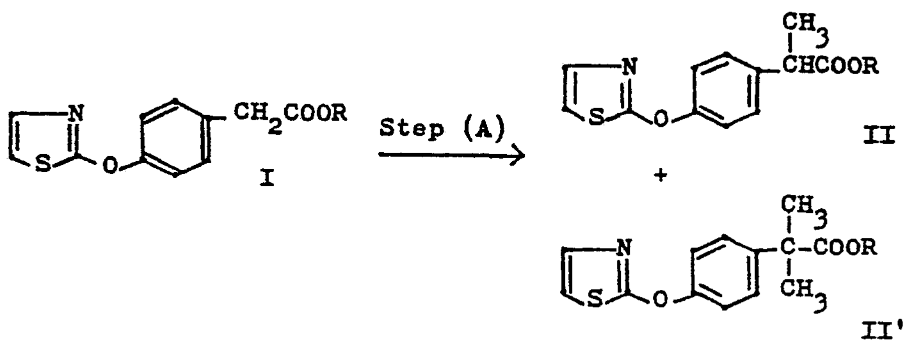


I

II

+

II'

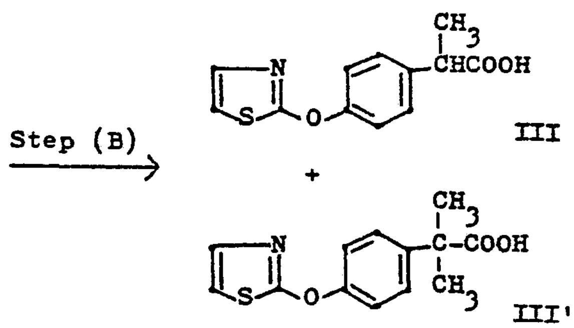


III

+

III'

STEP (A)

| Ex. No. | Comp. I R | Eq. mole of $CH_3Br/KOH$ | Reaction conditions Temp. (°C) | Time (hr) | Ratio (%) I:II:II′ |
|---|---|---|---|---|---|
| 2 | s-Butyl | 2.5/2.5 | 0 — −5 | 3.5 | 0:95.9:3.0 |
| 3 | t-Butyl | 2.7/2.7 | 0 — −5 | 5 | 0:96.3:3.4 |
| 4 | Benzhydryl | 2.0/2.0 | 0 — −5 | 3 | 0:93.5:4.7 |

STEP (B)

| Ex. No. | Eq. mole of NaOH | Ratio of MeOH/$H_2O$ | Reaction conditions Temp. (°C) | Time (hr) | Ratio (%) III:III' | Total Yield of III (1) → (2) |
|---|---|---|---|---|---|---|
| 2 | 4 | 3/1 | 27—30 | 4 | 99.7:0.17 | 90.1 |
| 3 | 5 | 3/1 | 50—55 | 6 | 99.9:0 | 82.0 |
| 4 | 2.5 | 3/1 | 24—25 | 2.5 | 99.6:0.2 | 89.5 |

Reference Preparation of isopropyl 2-[4-(2-thiazolyloxy)phenyl]acetate

To a solution of methyl 4-hydroxyphenylacetate (3.6 kg) in methanol (10.8 L) are added sodium hydroxide (2.0 g) and water (8 L). The mixture is allowed to react at 30°C for 1 hour and condensed under reduced pressure. To the residue is added water (3 L). The mixture is adjusted to pH 2.0 with conc. sulfuric acid and extracted with ethyl acetate (5 L). The extract is condensed under reduced pressure. To the resultant crystalline residue (3.3 kg) are added isopropanol (14 L) and conc. sulfuric acid (212 g). The mixture is refluxed for 5 hours and condensed under reduced pressure after cooling. Sodium hydrogencarbonate (500 g), water (8 L) and toluene (5 L) are added to the residue. The organic layer is condensed under reduced pressure and hexane (6 L) is added to the residue to precipitate isopropyl (6 L) is added to the residue to precipitate isopropyl 4-hydroxyphenylacetate (4.0 kg) as crystals melting at 36—38°C.

To an acetone solution (16 L) of the above product are added powdered potassium carbonate (5.7 kg) and 1-isobutoxy-2-chloroethyl isothiocyanate (4.4 kg). The mixture is allowed to react for 4 hours and then filtered. The filtrate is condensed under reduced pressure to give isopropyl 2-[4-(4-isobutoxy-2-thiazolin-2-yloxy)phenyl]acetate (8.0 kg). To a solution of the above product (8.0 kg) in dimethylformamide (28 L) is added p-toluenesulfonic acid (39 g) at 95°C. The mixture is heated for 15 minutes and condensed under reduced pressure after cooling.

To the residue is added a mixture of toluene (5 L) and water (50 L). The organic layer is washed with a 4% sodium hydroxide solution and filtered to remove the precipitate. The filtrate is washed with a 4% sodium hydroxide solution (5 L), 2% sulfuric acid (5 L) water (20 L) successively and filtered after addition of active carbon. The filtrate is condensed under reduced pressure to give oily isopropyl 2-[4-(2-thiazolyloxy)-phenyl]acetate (3.0 kg).

IRνCHCl$_3$ 1725cm$^{-1}$

NMR ($CDCl_3$) δ 1.23 (6H, d, J=7Hz), 3.60 (2H, s), 5.05 (1H, sep. J=7Hz), 6.83, 7.30 (2H, AB-type, q, J=4Hz), 7.32 (4H, m)

The following compounds are prepared by the same operation.

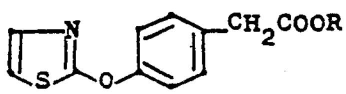

I

| Comp. I | Physical Constants | |
|---|---|---|
| R= | IR($CHCl_3$)cm$^{-1}$ | NMR$_{(CDCl_3)}$ δ$_{(=Hz)}$ |
| sec-Butyl (oil) | 1725 | 0.85 (3H, t, J=7) 1.18 (3H, d, J=7) 1.33—1.86 (2H, m) 3.60 (2H, s) 4.86 (1H, sex, J=7) 6.81, 7.23 (2H, ABq, J=4) 7.28 (4H, m) |
| tert-Butyl (mp 45°C) | 2980, 1725 1505, 1460 | 1.41 (9H, s) 3.50 (2H, s) 6.78, 7.23 (2H, ABq, J=4) 7.26 (4H, m) |
| Benzhydryl (mp 69°C) | 3000, 1735 1505, 1460 | 3.71 (2H, s) 6.73 (1H, d) 6.81 (1H, s) 7.20 (15H, m) |

**Claims**

1. A process for preparing 2-[4-(2-thiazolyloxy)phenyl]propionic acid which comprises condensing a compound of the formula:

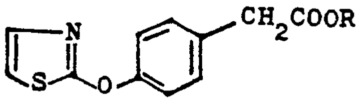

with methyl bromide, in dimethylformamide, in the presence of a condensing agent, to form a compound of the formula:

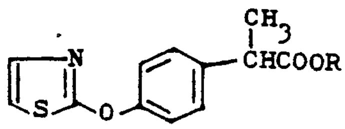

and hydrolysing this latter compound, with a base, in an aqueous inert organic solvent, characterized in that R is isopropyl, sec-butyl, tert-butyl or benzhydryl and in that the condensation is carried out with 1.0—3.0 equivalent moles of methyl bromide in dimethylformamide at a temperature of from −5 to 5°C in the presence of 1.0—3.0 equivalent moles of powdered potassium hydroxide, as a condensing agent, and in that the hydrolysis is carried out with 2—6 equivalent moles of sodium hydroxide at a temperature of from 0 to 70°C in aqueous methanol, ethanol or isopropanol.

2. The process claimed in claim 1 wherein 2.5—4.5 equivalent moles of sodium hydroxide are used at a temperature of 10—30°C in an aqueous methanol consisting of water and methanol at a ratio of 1:2—5 (v/v).

3. The process claimed in claim 1 wherein 4 equivalent moles of sodium hydroxide are used at a temperature of 15—25°C in an aqueous methanol consisting of water and methanol at a ratio of 1:3 (v/v).

4. The process claimed in claim 1 or 2 wherein 1.5—2.5 equivalent moles of methyl bromide and 1.5—2.5 equivalent moles of potassium hydroxide are used at a temperature of 0—5°C.

5. The process claimed in claim 1 or 3 wherein 2.0—2.5 equivalent moles of methyl bromide and 2.0—2.5 equivalent moles of potassium hydroxide are used at a temperature of 0—5°C.

6. The process claimed in any one of claims 1—5 wherein R is isopropyl, sec-butyl or benzhydryl.

7. The process claimed in any one of claims 1—5 wherein R is isopropyl.

**Patentansprüche**

1. Verfahren zur Herstellung von 2-[4-(2-Thiazolyloxy)phenyl]-propionsäure, das die Kondensierung einer Verbindung der Formel

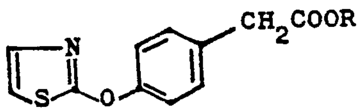

mit Methylbromid in Dimethylformamid in Anwesenheit eines Kondensierungsmittels unter Bildung einer Verbindung der Formel

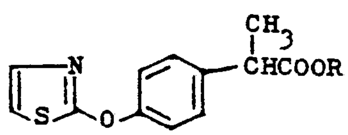

und die Hydrolyse dieser zweiten Verbindung mit einer Base in einem wässrigen inerten organischen Lösungsmittel umfaßt, dadurch gekennzeichnet, daß R eine Isopropyl-, sek.Butyl-, tert.Butyl- oder Benzhydrylgruppe ist, und daß die Kondensierung mit 1,0 bis 3,0 Moläquivalenten Methylbromid in Dimethylformamid bei einer Temperatur von −5°C bis 5°C in Anwesenheit von 1,0 bis 3,0 Moläquivalenten gepulvertem Kaliumhydroxid als Kondensierungsmittel durchgeführt wird, und daß die Hydrolyse mit 2—6 Moläquivalenten Natriumhydroxid bei einer Temperatur von 0 bis 70°C in wässrigem Methanol, Äthanol oder Isopropanol durchgeführt wird.

2. Verfahren nach Anspruch 1, wobei 2,5 bis 4,5 Moläquivalente Natriumhydroxid bei einer Temperatur von 10—30°C in wässrigem Methanol, das aus Wasser und Methanol in einem Verhältnis von 1:2—5 (v/v) besteht, verwendet werden.

3. Verfahren nach Anspruch 1, wobei 4 Moläquivalente Natriumhydroxid bei einer Temperatur von 15—25°C in wässrigem Methanol, das aus Methanol in einem Verhältnis von 1:3 (v/v) besteht, verwendet werden.

4. Verfahren nach Anspruch 1 oder 2, wobei 1,5—2,5 Moläquivalente Methylbromid und 1,5—2,5 Moläquivalente Kaliumhydroxid bei einer Temperatur von 0—5°C verwendet werden.

5. Verfahren nach Anspruch 1 oder 3, wobei 2,0—2,5 Moläquivalente Methylbromid und 2,0—2,5 Moläquivalente Kaliumhydroxid bei einer Temperatur von 0—5°C verwendet werden.

6. Verfahren nach einem der Ansprüche 1—5, wobei R eine Isopropyl-, sek.Butyl- oder Benzhydrylgruppe ist.

7. Verfahren nach einem der Ansprüche 1—5, wobei R eine Isopropylgruppe ist.

**Revendications**

1. Procédé de préparation de l'acide 2-(4-(2-thiazolyloxy)phényl)-propionique, qui consiste à condenser un composé répondant à la formule:

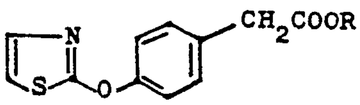

avec du bromure de méthyle, dans du diméthylformamide, en présence d'un agent de condensation, pour former un composé répondant à la formule:

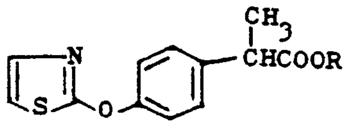

et à hydrolyser ce dernier composé avec un base, dans un solvant organique inerte aqueux, caractérisé en ce que R est un groupe isopropyle, sec-butyle, tert-butyle ou benzhydryle et en ce que la condensation est effectuée avec 1,0 à 3,0 équivalents molaires de bromure de méthyle dans du diméthylformamide à une température de −5 à 5°C en présence de 1,0 à 3,0 équivalents molaires d'hydroxyde de potassium pulvérisé, comme agent de condensation, et en ce que l'hydrolyse est effectuée avec 2 à 6 équivalents molaires d'hydroxyde de sodium à une température de 0 à 70°C dans du méthanol, de l'éthanol ou de l'isopropanol aqueux.

2. Procédé selon la revendication 1, dans lequel on utilise 2,5 à 4,5 équivalents molaires d'hydroxyde de sodium à une température de 10 à 30°C dans un méthanol aqueux constitué d'eau et de méthanol dans un rapport de 1:2—5 (V/V).

3. Procédé selon la revendication 1, dans lequel on utilise 4 équivalents molaires d'hydroxyde de sodium à une température de 15 à 25°C dans un méthanol aqueux constitué d'eau et de méthanol dans un rapport de 1:3 (V/V).

4. Procédé suivant les revendications 1 ou 2, dans lequel on utilise 1,5 à 2,5 équivalents molaires de bromure de méthyle et 1,5 à 2,5 équivalents molaires d'hydroxyde de potassium à une température de 0 à 5°C.

5. Procédé suivant les revendications 1 ou 3, dans lequel on utilise 2,0 à 2,5 équivalents molaires de bromure de méthyle et 2,0 à 2,5 équivalents molaires d'hydroxyde de potassium à une température de 0 à 5°C.

6. Procédé suivant l'une quelconque des revendications 1 à 5, dans lequel R est un groupe isopropyle, sec-butyle ou benzhydryle.

7. Procédé suivant l'une quelconque des revendications 1 à 5, dans lequel R est un groupe isopropyle.